(19) Europäisches Patentamt
European Patent Office
Office européen des brevets

(11) Numéro de publication: **0 182 768**
**A1**

(12) **DEMANDE DE BREVET EUROPEEN**

(21) Numéro de dépôt: **85870149.3**

(22) Date de dépôt: **04.11.85**

(51) Int. Cl.[4]: **C 07 D 233/84**, C 07 D 235/28, C 07 D 263/58, A 61 K 31/415, A 61 K 31/42

(30) Priorité: **13.11.84 FR 8417286**

(43) Date de publication de la demande: **28.05.86 Bulletin 86/22**

(84) Etats contractants désignés: **AT BE CH DE FR GB IT LI LU NL SE**

(71) Demandeur: **SANOFI, 40, Avenue George V, F-75008 Paris (FR)**
Demandeur: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Sebille, Bernard, rue Pierre Louvrier 45bis, F-92140 Clamart (FR)**
Inventeur: **Beuzard, Yves, rue de Villersexel 7, F-75008 Paris (FR)**
Inventeur: **Demarne, Henri, Avenue du Major Flandre 65, F-34100 Montpellier (FR)**

(74) Mandataire: **Cauchie, Daniel, c/o S.A. LABAZ-SANOFI N.V. Avenue De Béjar, 1, B-1120 Bruxelles (BE)**

(54) **Compositions pharmaceutiques ou vétérinaires contenant des dérivés thiosulfonates.**

(57) L'invention se rapporte à des compositions pharmaceutiques ou vétérinaires destinées à inhiber la malformation ou la destruction des érythrocytes due à une modification génétique de l'hémoglobine ou à des parasites, compositions contenant, comme principe actif essentiel, au moins un dérivé thiosulfonate de formule générale:

$$R-S-SO_2-R_1$$

dans laquelle R représente un radical méthyl-1 imidazolyl-2, 1H-benzimidazolyl-2 ou benzoxazolyl-2 et $R_1$ représente un radical alkyle inférieur, (hydroxy) alkyle inférieur, alcanoyle inférieur, phényle, (alkyle inférieur) phényle, (hydroxy alkyle inférieur) phényle, (alcanoyle inférieur) phényle ou quinoléinyl-8.

Les compositions de l'invention sont utiles pour le traitement de la drépanocytose, du paludisme et de la babésiose.

EP 0 182 768 A1

# COMPOSITIONS PHARMACEUTIQUES OU VETERINAIRES CONTENANT DES DERIVES THIOSULFONATES.

La présente invention se rapporte d'une manière générale, à des compositions pharmaceutiques ou vétérinaires destinées notamment à inhiber la malformation ou la destruction des érythrocytes due à une modification génétique de l'hémoglobine ou à des parasites.

En particulier, l'invention concerne des compositions pharmaceutiques ou vétérinaires contenant, comme principe actif essentiel, au moins un dérivé thiosulfonate de formule générale :

$$R\text{-}S\text{-}SO_2\text{-}R_1 \qquad I$$

dans laquelle R représente un radical méthyl-1 imidazolyl-2, 1H-benzimidazolyl-2 ou benzoxazolyl-2 et $R_1$ représente un radical alkyle inférieur, (hydroxy) alkyle inférieur, alcanoyle inférieur, phényle, (alkyle inférieur) phényle, (hydroxy alkyle inférieur) phényle, (alcanoyle inférieur) phényle ou quinoléinyl-8.

Par "alkyle inférieur", on entend plus particulièrement les groupements méthyle, éthyle, n-propyle et isopropyle et par "alcanoyle inférieur" on désigne plus spécialement les groupements formyle, acétyle et propionyle.

Il est connu que la drépanocytose est une maladie génétique qui comporte une anomalie de la structure de l'hémoglobine. Cette anomalie est le remplacement de l'acide aminé Glu-6 de la chaîne β par l'acide aminé Val pour donner l'hémoglobine S qui polymérise.

Cette polymérisation, lors de la désoxygénation du globule rouge, entraîne la falciformation de ce dernier qui devient rigide, circule mal et se bloque dans les petits vaisseaux. Les individus porteurs de deux gènes drépanocytaires sont ainsi sous la menace permanente d'une complication fatale.

Certains disulfures ont été décrits dans la littérature comme inhibant la falciformation, notamment la cystamine qui se révèle particulièrement active ("Developments of Therapeutic Agents for Sickle Cell Disease", Inserm Symposium, 1979, North Holland : Amsterdam, Editeurs J. ROSA, Y. BEUZARD, J. HERCULES; p. 139-153).

Par contre, on ne connaît, à l'heure actuelle, aucun dérivé thiosulfonate

présentant une telle activité inhibitrice de la falciformation.

On a maintenant trouvé que des dérivés thiosulfonates, en l'occurrence les composés de formule I ci-dessus présentent une activité importante vis-à-vis de la polymérisation de l'hémoglobine S et de la falciformation des globules rouges.

On a également trouvé, de manière surprenante, que les dérivés thiosulfonates de formule I exercent une activité sur les parasites affectant les érythrocytes tels que les Plasmodia et les Babesiae. En particulier, les composés de formule I ont montré une activité schizonticide in vitro sur Plasmodium falciparum et in vivo sur Plasmodium berghei.

L'expression "inhibition de la malformation ou de la destruction des érythrocytes" utilisée ci-dessus signifie une inhibition qui peut être soit directe sur l'hémoglobine soit indirecte par inhibition de la croissance des parasites dans le globule rouge.

En outre, les composés de formule I ont révélé un degré de toxicité relativement faible et en tout état de cause compatible avec une utilisation à des fins thérapeutiques.

En conséquence, les composés de formule I constituent de très intéressants agents inhibiteurs de la malformation ou de la destruction des érythrocytes due à une modification génétique de l'hémoglobine ou à des parasites et, à ce titre, seront utiles dans le traitement de la drépanocytose, du paludisme et de la babébiose.

L'inhibition de la falciformation des érythrocytes humains a été évaluée selon une technique consistant à laver et à incuber, dans un tampon salin, (pH : 7,40 ; 0,15M), les globules rouges de sujets atteints de drépanocytose pendant une heure à 37°C. Cette opération est effectuée dans un bain-marie, sous agitation circulaire et en présence d'un composé de formule I utilisé à différentes concentrations. On teste ainsi initialement le rapport molaire composé de formule I/hémoglobine dans l'intervalle allant de 0,5 à 20 (concentration hémoglobine : 0,5mM) et en fonction des résultats obtenus, on modifie ce rapport.

A la fin de l'incubation, on enlève par lavage l'excès de composé à étudier et on transfère dans un Erlenmeyer la suspension cellulaire ajustée à un hématocrite de 5%. On incube alors à 37°C sous un courant d'un mélange humidifié d'azote et d'oxygène, la concentration d'oxygène étant réglée par une pompe de mélange gazeux. On transfère le gaz effluent dans un autre tube

contenant du formaldéhyde et, en fin d'incubation, on introduit la suspension cellulaire dans le formaldéhyde par simple retournement de la fiole.

On évalue alors la proportion de cellules déformées et celles ayant les caractéristiques de drépanocytes (ayant deux prolongements filiformes) au moyen d'un microscope ayant une optique interférentielle de NOMARSKY. On calcule l'inhibition de la falciformation selon la formule suivante :

$$\frac{\text{\% cellules falciformées du témoin} - \text{\% cellules falciformées en présence du composé de formule I}}{\text{\% cellules falciformées du témoin}}$$

On a obtenu les résultats suivants avec un composé représentatif de formule I en comparaison avec la cystamine. Ces résultats représentent les pourcentages d'inhibition de la falciformation des érythrocytes.

| Composé | Concentration (mmolaire) | % inhibition |
|---|---|---|
| p-Toluènethiosulfonate de benzoxazolyl-2 | 5 | 100 |
| Cystamine | 5 | 33 |

Ces résultats montrent que le composé de formule I exerce une inhibition de la falciformation des érythrocytes des drépanocytaires et est plus actif que le composé de comparaison.

Les composés de formule I ci-dessus sont des composés connus ayant été publiés dans les demandes de brevet japonais Nos. 81-99335 (C.A. 96, 113510s) et 82-140187 (C.A. 99, 222446g) à titre de produits photothermographiques sans qu'aucune activité pharmacologique ou utilisation thérapeutique ne soit mentionnée.

Ces composés de formule I peuvent être préparés, notamment, en faisant réagir, en présence d'un accepteur d'acide tel que par exemple la pyridine, un dérivé mercapto de formule générale :

R-SH II

dans laquelle R a la même signification que précédemment, avec un dérivé halogénosulfonyle de formule générale :

$$Hal\text{-}SO_2\text{-}R_1 \qquad \text{III}$$

dans laquelle $R_1$ a la même signification que précédemment et Hal représente un atome d'halogène de préférence le chlore.

La réaction peut être effectuée dans un solvant organique approprié tel que par exemple le dichlorométhane ou l'acétonitrile.

Le rapport molaire dérivé mercapto de formule II/dérivé halogénosulfonyle de formule III joue un rôle essentiel, de même que la température, dans le rendement en dérivé thiosulfonate de formule I.

Ainsi, à la température ambiante et selon un rapport molaire : $\frac{\text{composé de formule II}}{\text{composé de formule III}} = 2$, on obtient essentiellement les disulfures de formule R-S-S-R, dans laquelle R a la même signification que précédemment.

Par exemple, 90% de dithio-2,2' di-1H-benzimidazole sont produits à la température ambiante à partir de mercapto-2 1H-benzimidazole et de chlorure de benzènesulfonyle dans le rapport molaire 2:1.

Cependant, en contrôlant la température et en modifiant la proportion molaire des réactifs de formules II et III, on obtient préférentiellement les dérivés thiosulfonates de formule I.

On effectue, en conséquence le procédé décrit ci-dessus à une température de 0 à 10°C, généralement à la température de l'eau glacée, le rapport molaire $\frac{\text{composé de formule II}}{\text{composé de formule III}}$ pouvant varier de 1:1 à 1:4. En général, on préfère un rapport molaire 1:2.

Les composés de formule I peuvent être administrés en tant que principe actif médicamenteux sous forme de compositions unitaires convenant à l'administration en thérapie humaine ou vétérinaire notamment pour l'administration orale, sublinguale, sous-cutanée, intramusculaire, intraveineuse, transdermique ou rectale.

Pour ce qui concerne l'unité d'administration, celle-ci peut prendre la forme par exemple, d'un comprimé, d'une dragée, d'une capsule, d'une gélule, d'une poudre, d'une suspension ou d'un sirop pour l'administration orale, d'un suppositoire pour l'administration rectale, d'une solution stérile ou suspension pour l'administration parentérale.

La quantité de principe actif en question est de 10 à 1000 mg par unité d'administration selon que cette unité est destinée à l'administration orale, rectale ou parentérale. Généralement une unité d'administration sera nécessaire 1 à 4 fois par jour de manière que la quantité journalière de principe actif selon l'invention puisse varier de 0,1 à 100 mg par kg de poids corporel pour le traitement de la drépanocytose, du paludisme et de la babébiose.

Sous leur aspect le plus général et en tenant compte de la voie d'administration choisie, les compositions de l'invention sont préparées en associant au moins un dérivé thiosulfonate de formule I ci-dessus, avec un véhicule pharmaceutique ou un excipient approprié, ce dernier pouvant être sélectionné parmi des substances telles que l'eau distillée, l'alcool benzylique, le lactose, les amidons, le talc, le stéarate de magnésium, la polyvinylpyrrolidone, l'acide alginique, la silice colloïdale, la cellulose microcristalline, des esters phtaliques ou acryliques, le dioxyde de titane, les agents édulcorants,etc...

On décrit par la suite, de manière plus particulière mais non limitative, quelques modes de réalisation de compositions selon l'invention.

On prépare, par exemple, des poudres pour l'administration orale en broyant simplement le principe actif à une finesse convenable et en mélangeant avec un diluant broyé de manière semblable, lequel peut être par exemple un composé glucidique comestible, tel que l'amidon.

Avantageusement, on incorpore un agent édulcorant ou un sucre ainsi qu'une huile aromatisante.

On peut préparer les granulés destinés à la reconstitution d'une préparation liquide orale en mouillant le composé actif de formule I et un diluant soluble dans l'eau tel que saccharose, glucose, etc... avec un liant tel que mucilage d'acacia, solution de gélatine, solution de méthylcellulose.

On fait alors passer ce mélange pulvérulent sur un tamis en forçant pour former des granulés que l'on sèche. De manière avantageuse, on introduit, dans la composition, un agent de mise en suspension comme la gomme adragante. De même, on obtient une composition de l'invention sous forme de gélule en introduisant, dans des gaines de gélatine molles ou dures, un mélange pulvérulent décrit ci-dessus.

Comme adjuvant à l'opération de remplissage, il est avantageux d'ajouter un lubrifiant tel que talc, stéarate de magnésium ou stéarate de calcium au mélange pulvérulent.

On peut également obtenir des composés en formant d'abord un mélange pulvérulent à partir du composé actif de formule I, convenablement broyé et d'un diluant ou d'une base telle qu'amidon, saccharose, kaolin, phosphate bicalcique etc... puis en granulant ou en tronçonnant après addition d'un lubrifiant et finalement en comprimant. La granulation du mélange pulvérulent peut se faire en mouillant avec un liant tel que sirop, pâte d'amidon ou mucilage d'acacia et en forçant au travers d'un tamis.

Une autre méthode de granulation consiste à tronçonner le mélange pulvérulent en le faisant passer au travers d'une machine à comprimés puis à casser en fragments (tronçons) les comprimés résultant imparfaitement formés. On peut lubrifier les tronçons pour les empêcher de coller en formant des cubes par addition d'un sel stéarate, de talc ou d'une huile minérale. On comprime ensuite le mélange lubrifié pour constituer les comprimés définitifs. Si nécessaire, les comprimés obtenus peuvent être dragéifiés, enrobés d'un vernis à délitage entérique ou encore enrobés de telle manière que le principe actif se libère graduellement.

On peut préparer, également, une composition pour administration par voie rectale sous forme de suppositoire en coulant, dans un moule approprié, un mélange formé du principe actif ci-dessus et d'un agent liant fondant à la température rectale par exemple beurre de cacao, polyéthylèneglycols ou lanoline.

Pour une administration parentérale, on réalise des suspensions aqueuses, des solutions salines isotoniques ou des solutions stériles et injectables contenant le composé actif de formule I ainsi qu'un agent de solubilisation si nécessaire, par exemple le polysorbate 80, des agents mouillants appropriés par exemple le propylèneglycol ou le butylèneglycol et un agent de conservation tel que l'alcool benzylique.

Le principe actif peut être formulé également sous forme de microcapsules éventuellement avec un ou plusieurs additifs.

A titre d'Exemples non limitatifs, on décrira, par la suite, la préparation des composés de formule I ainsi que des compositions les contenant.

EXEMPLE 1

Préparation du benzènethiosulfonate de 1H-benzimidazolyl-2

Dans un ballon tricol de 500 ml équipé d'un réfrigérant et d'un agitateur magnétique, on dissout 3,51 g (0,02 mole) de chlorure de benzènesulfonyle

dans 40 ml de dichlorométhane tout en maintenant une agitation suffisamment efficace. A cette solution, on ajoute 3,16 g (0,04 mole) de pyridine sèchée au préalable sur du sulfate de magnésium.

On plonge le ballon dans un bain d'eau glacée à 4°C et dans le mélange réactionnel maintenu sous agitation à la température de l'eau glacée, on ajoute lentement (temps d'addition : > 3 heures) et goutte à goutte à l'aide d'une ampoule à addition, 1,5 g (0,01 mole) de mercapto-2 1H-benzimidazole dissous dans 160 ml de dichlorométhane.

On laisse le mélange réactionnel sous agitation vigoureuse durant 24 heures à 4°C. Un précipité de couleur crème apparaît progressivement dans le ballon. Pour faciliter la formation et la récupération du solide, on évapore une partie du solvant sous vide en chauffant très légèrement (environ 50°C). On récupère le précipité par filtration sur verre fritté et on le sèche. On lave 3 fois avec de l'éthanol et on recristallise dans le même solvant.

On obtient de cette manière, le benzènethiosulfonate de 1H-benzimidazolyl-2.

Rendement : 80%

Formule brute : $C_{13}H_{10}N_2O_2S_2$

Analyse :

| | C% | H% | N% | O% | S% |
|---|---|---|---|---|---|
| Calculé | 53,78 | 3,47 | 9,65 | 11,02 | 22,08 |
| Trouvé | 53,71 | 3,51 | 10,19 | 11,60 | 21,69 |

P.F. : 201°C

De la même manière que celle décrite précédemment, on a obtenu les composés suivants :

a) à partir de chlorure de p-toluènesulfonyle et de mercapto-2 1H-benzimidazole, le p-toluènethiosulfonate de 1H-benzimidazolyl-2.

Rendement : 70%

Formule brute : $C_{14}H_{12}N_2O_2S_2$

Analyse :

| | C% | H% | N% | O% | S% |
|---|---|---|---|---|---|
| Calculé | 55,24 | 3,97 | 9,20 | 10,51 | 21,07 |
| Trouvé | 55,24 | 3,92 | 9,19 | 10,46 | 21,07 |

P.F. : 169°C

b) à partir de chlorure de benzènesulfonyle et de méthyl-1 mercapto-2 1H-imidazole, le benzènethiosulfonate de méthyl-1 1H-imidazolyl-2.

Rendement : 45%

P.F. : 84°C

Formule brute : $C_{10}H_{10}N_2O_2S_2$

Analyse :

| | C% | H% | N% | O% | S% |
|---|---|---|---|---|---|
| Calculé | 47,23 | 3,96 | 11,01 | 12,58 | 25,21 |
| Trouvé | 46,98 | 4,37 | 11,07 | 12,77 | 24,81 |

c) à partir de chlorure de p-toluènesulfonyle et de mercapto-2 benzoxazole, le p-toluènethiosulfonate de benzoxazolyl-2.

Rendement : 75%

P.F. : 93°C

Formule brute : $C_{14}H_{11}NO_3S_2$

Analyse :

| | C% | H% | N% | O% | S% |
|---|---|---|---|---|---|
| Calculé | 55,07 | 3,63 | 4,59 | 15,72 | 21,00 |
| Trouvé | 54,49 | 3,61 | 5,03 | 15,91 | 20,95 |

d) à partir de chlorure de méthanesulfonyle et de mercapto-2 1H-benzimidazole, le méthanethiosulfonate de 1H-benzimidazolyl-2.

Rendement : 50%

P.F. : 157°C

Formule brute : $C_8H_8N_2O_2S_2$

Analyse :

| | C% | H% | N% | O% | S% |
|---|---|---|---|---|---|
| Calculé | 42,09 | 3,53 | 12,27 | 14,02 | 28,09 |
| Trouvé | 42,05 | 3,62 | 12,17 | 14,61 | 27,47 |

EXEMPLE 2

On prépare des comprimés renfermant, en tant que principe actif, un dérivé thiosulfonate de formule I, associé à un excipient pour former la composition ci-dessous :

| | mg |
|---|---|
| Composé de formule I | 500 |
| Glycine | 120 |
| Cellulose microcristalline | 70 |
| Silice précipitée | 18 |
| Carboxyméthylamidon | 30 |
| Stéarate de magnésium | 11 |
| Talc | 11 |

On mélange pendant 30 min. les ingrédients de la composition ci-dessus, puis on granule à sec et on fait passer le mélange à travers un tamis à mailles de 1,6 mm.

On comprime ensuite en utilisant un poinçon ayant la forme d'un bâtonnet. On obtient ainsi des comprimés pesant chacun 760 mg et renfermant chacun 500 mg de principe actif.

## EXEMPLE 3

On prépare des comprimés dragéifiés en utilisant les comprimés obtenus à l'Exemple 2 et en les enrobant à l'aide d'une suspension de phtalate de dibutyle, de polyméthacrylate de butyle et diméthylaminoéthyle, de polyéthylèneglycol 1500, de silice précipitée, de dioxyde de titane et de talc dans un mélange acétone/isopropanol 1/1 ayant une concentration en résidu sec de 10% environ.

On obtient ainsi des comprimés dragéifiés pesant chacun 780mg et renfermant chacun 500 mg de principe actif.

## EXEMPLE 4

On prépare un granulé destiné à la reconstitution d'une préparation liquide pour administration orale renfermant, en tant que principe actif, un dérivé thiosulfonate de formule I associé à un excipient pour former la composition suivante :

| | g |
|---|---|
| Composé de formule I | 3,60 |
| Saccharose | 50,00 |
| Carboxyméthylcellulose sodique | 0,80 |
| Acide citrique | 0,10 |
| Citrate trisodique | 0,90 |

| | |
|---|---|
| Benzoate de sodium | 0,25 |
| Saccharine sodique | 0,15 |
| Aromatisant | 0,50 |

On pulvérise les composants ci-dessus à l'exception du saccharose puis on mélange la poudre ainsi obtenue avec le saccharose jusqu'à obtenir un granulé homogène.

On porte alors le volume du granulé ainsi obtenu à 100 ml avec de l'eau destinée à la préparation de sirops. Une dose unitaire de 5 ml du sirop extemporané ainsi obtenu contient 180 mg de principe actif.

## EXEMPLE 5

On prépare selon l'Exemple 4 un granulé destiné à la reconstitution d'une préparation liquide orale ayant la composition suivante :

| | g |
|---|---|
| Composé de formule I | 7,00 |
| Saccharose | 46,60 |
| Carboxyméthylcellulose sodique | 0,90 |
| Acide citrique | 0,10 |
| Citrate trisodique | 0,90 |
| Benzoate de sodium | 0,25 |
| Saccharine sodique | 0,15 |
| Aromatisant | 0,50 |

Une dose unitaire de 5 ml du sirop extemporané ainsi obtenu contient 350 mg de principe actif.

## EXEMPLE 6

On prépare selon l'Exemple 4 un granulé destiné à la reconstitution d'une préparation liquide orale ayant la composition suivante :

| | g |
|---|---|
| Composé de formule I | 8,00 |
| Saccharose | 45,60 |
| Carboxyméthylcellulose sodique | 1,00 |
| Acide citrique | 0,10 |
| Citrate trisodique | 0,90 |
| Benzoate de sodium | 0,25 |
| Saccharine sodique | 0,15 |
| Aromatisant | 0,50 |

Une dose unitaire de 5 ml du sirop extemporané ainsi obtenu contient 400 mg de principe actif.

EXEMPLE 7

On prépare des comprimés contenant un composé décrit dans l'Exemple 1, comprimés ayant la composition suivante :

| | mg |
|---|---|
| Composé de formule I | 350 |
| Cellulose microcristalline | 100 |
| Lactose | 125 |
| Stéarate de magnésium | 10 |
| Talc | 15 |
| | 600 |

On fait passer les poudres à travers un tamis de 0,3 mm, puis on mélange les ingrédients jusqu'à obtenir un mélange homogène que l'on comprime puis granule.

Les granulés ainsi obtenus sont utilisés pour former des comprimés par compression.

EXEMPLE 8

On prépare des comprimés contenant un composé décrit dans l'Exemple 1, comprimés ayant la composition suivante :

| | mg |
|---|---|
| Composé de formule I | 150 |
| Cellulose microcristalline | 75 |
| Talc | 15 |
| Polyvinylpyrrolidone | 30 |
| Silice précipitée | 25 |
| Stéarate de magnésium | 5 |
| | 300 |

On mélange intimement et pendant 15 min. dans une mélangeuse-pétrisseuse tous les ingrédients sauf le lubrifiant puis on pétrit par addition graduelle d'eau. On fait passer la masse à travers un tamis de 1,25 mm puis on sèche le granulé dans une étuve à ventilation forcée jusqu'à obtenir une humidité résiduelle relativement réduite (environ 2%). On uniformise le granulé, on ajoute le lubrifiant et on forme des comprimés par compression.

De la même manière que précédemment, on prépare des comprimés contenant 250 mg de principe actif de formule I.

EXEMPLE 9

On prépare des comprimés enrobés contenant un composé décrit dans l'Exemple 1, comprimés ayant la composition suivante :

| | mg |
|---|---|
| Composé de formule I | 150 |
| Carboxyméthylamidon | 10 |
| Cellulose microcristalline | 85 |
| Lactose | 135 |
| Huile de ricin hydrogénée | 10 |
| Stéarate de magnésium | 5 |

en opérant comme décrit à l'Exemple 8.

On recouvre les comprimés ainsi obtenus par un revêtement ayant la composition suivante :

| | mg |
|---|---|
| Phtalate de butyle | 0,300 |
| Polyméthacrylate de butyle et diméthylaminoéthyle | 1,850 |
| Polyéthylèneglycol 1500 | 0,080 |
| Silice précipitée | 0,020 |
| Talc | 0,900 |
| Dioxyde de titane | 1,850 |

Cette composition est dissoute dans un solvant qui est éliminé par évaporation dans une étuve à ventilation forcée.
Poids d'un comprimé : 400 mg.

EXEMPLE 10

On prépare des suppositoires contenant un composé décrit dans l'Exemple 1, suppositoires ayant la composition suivante :

| | mg |
|---|---|
| Composé de formule I | 300 |
| Masse pour suppositoires | 1.450 |
| | 1.750 |

On met en suspension à 37°C, la substance active finement pulvérisée dans la masse pour suppositoires et on verse le mélange dans des moules légèrement refroidis au préalable.

REVENDICATIONS

1. Compositions pharmaceutiques ou vétérinaires caractérisées en ce qu'elles contiennent, comme principe actif, au moins un dérivé thiosulfonate de formule générale :

$$R\text{-}S\text{-}SO_2\text{-}R_1$$

dans laquelle R représente un radical méthyl-1 imidazolyl-2, 1H-benzimidazolyl-2 ou benzoxazolyl-2 et $R_1$ représente un radical alkyle inférieur, (hydroxy) alkyle inférieur, alcanoyle inférieur, phényle, (alkyle inférieur) phényle, (hydroxy alkyle inférieur) phényle, (alcanoyle inférieur) phényle ou quinoléinyl-8, en association avec un véhicule pharmaceutique ou un excipient approprié.

2. Compositions selon la Revendication 1 destinées à inhiber la malformation ou la destruction des érythrocytes due à une modification génétique de l'hémoglobine ou à des parasites.

3. Compositions selon une des Revendications 1 et 2 caractérisées en ce qu'elles contiennent le p-toluenethiosulfonate de benzoxazolyl-2.

4. Compositions selon une des Revendications 1 à 3 caractérisées en ce qu'elles sont sous forme d'unité de dosage.

5. Compositions selon une des Revendications 1 à 3 caractérisées en ce que chaque unité de dosage contient de 10 à 1000 mg de principe actif.

Office européen des brevets

0182768

Numero de la demande

# RAPPORT DE RECHERCHE EUROPEENNE

EP 85 87 0149

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 82, no. 17, 28 avril 1975, page 507, abrégé 111998u, Columbus, Ohio, US; S.I. ZAV'YALOV et al.: "Synthesis and some transformations of tosyl derivatives of 2-imidazolinone and 2-imidazolinethione" & IZV. AKAD. NAUK SSSR, SER. KHIM. 1974, (12), 2778-81<br>--- | | C 07 D 233/84<br>C 07 D 235/28<br>C 07 D 263/58<br>A 61 K 31/415<br>A 61 K 31/42 |
| A | CHEMICAL ABSTRACTS, vol. 71, no. 11, 15 septembre 1969, page 383, abrégé 49830a, Columbus, Ohio, US; Y. USUI: "Fungicides. XIV. Syntheses of 4,5,6,7-tetrahydrobenzothiazole-2-thiol derivatives" & YAKUGAKU ZASSHI 1968, 88(12), 1535-44<br>--- | | DOMAINES TECHNIQUES RECHERCHES (Int. Cl.4) |
| A | CHEMICAL ABSTRACTS, vol. 92, no. 3, 21 janvier 1980, page 677, abrégé 22420p, Columbus, Ohio, US; A.M. OSMAN et al.: "Synthesis of some new arylnaphthoxazoles: sulfonamides, sulfonic esters and thiosulfonic esters. Part III" & J. INDIAN. CHEM. SOC. 1979, 56(3), 293-6<br>--- -/- | | C 07 D 233/00<br>C 07 D 235/00<br>C 07 D 263/00<br>A 61 K 31/00 |

Le present rapport de recherche a été établi pour toutes les revendications

| Lieu de la recherche | Date d'achèvement de la recherche | Examinateur |
|---|---|---|
| LA HAYE | 20-12-1985 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

**RAPPORT DE RECHERCHE EUROPEENNE**

0182768

Numero de la demande

EP 85 87 0149



## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| D,A | CHEMICAL ABSTRACTS, vol. 96, no. 14, 5 avril 1982, page 637, abrégé 113510s, Columbus, Ohio, US; & JP - A - 81 99 335 (ORIENTAL PHOTO INDUSTRIAL CO., LTD.) 10.08.1981<br>--- | | |
| A | US-A-3 786 063 (ARNOLD)<br>--- | | |
| A | DE-A-2 151 895 (BAYER)<br>----- | | |
| | | | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 20-12-1985 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant